# EUROPEAN PATENT SPECIFICATION

(11) **EP 3 669 914 B1**
(45) Date of publication and mention of the grant of the patent: **30.08.2023**
(21) Application number: 19213935.0
(22) Date of filing: 05.12.2019
(51) Int. Cl.: A61M 5/32

(54) **INTRODUCER NEEDLE DEVICE**
EINFÜHR NADELVORRICHTUNG
DISPOSITIF D'AIGUILLE D'INTRODUCTEUR

(30) Priority: 17.12.2018 GB 201820526
(43) Date of publication of application: 24.06.2020
(73) Proprietor: Kimal PLC, Uxbridge Middlesex UB8 2SA (GB)
(72) Inventor: SHEWARD, Adam Nicholas, Uxbridge, Middlesex UB8 2SA (GB)
(74) Representative: Bazant-Hegemark, Florian

(56) References cited:
- WO-A2-02/066093
- US-A1- 2009 118 676
- US-A1- 2012 029 465
- US-A1- 2017 224 962

## Description

This invention relates generally to an introducer needle device and in particular, but not exclusively, to a guidewire introducer assembly.

### BACKGROUND

Introducer needle devices are typically elongate rigid needles that can be used to assist with the placement of another generally more flexible device, such as a guidewire or a catheter, into a part of the body or blood vessel. That is, introducer needle devices are most commonly used as a means of access for other medical devices into a part of the body or blood vessel. Typically, an introducer needle device will comprise an elongate needle having a tapered or bevelled tip at one end and a bore that can facilitate the passage of the medical device (e.g. a guidewire) into the body.

In some examples, an introducer needle device may further comprise a rigid cap or housing that surrounds at least the sharp bevelled end of the needle. The cap or housing is removed prior to use but serves to offer some protection to an end user from needle stick injuries, at least whilst the cap or housing is in place.

By way of a more specific example, US 2017 224962 A1, shows a cannular introducer assembly, referred to as a "catheter introducer assembly" comprising an outer tube, an outer tube housing and a needle. The needle is positioned within the outer tube and is movable within the outer tube from a first position to a second position upon coupling of a male luer slip of a syringe barrel to a female luer hub (retainer portion) of the needle. In the first position, the needle is retracted within the outer tube housing, whereas in the second position, the needle is exposed. When the male and female luer fittings are decoupled, the needle moves from the second, exposed position to the first, retracted position, to reduce the likelihood of needle stick injuries.

A problem with this and other prior art arrangements is that the position of a bevel of the needle within the housing is unknown. This is problematic because an end user, such as a clinician, may wish to use the introducer needle device in a particular orientation, for example in a "bevel down" or a "bevel up" position.

In an attempt to overcome this problem, it has become industry practice to provide a needle bevel indicator, often in the form of an arrow, on the external face of the introducer needle housing to indicate the position of the bevel therein. However, a problem with this solution is that it is possible for the needle to rotate coaxially within the housing, thus meaning that the needle bevel indicator may not necessarily indicate the accurate position of the bevel.

To further contribute to the aforementioned ambiguity, it is often not apparent whether or not the needle has in fact rotated from its original position as assembled. This means that in some cases, the indicator will accurately show the position of the bevel and in other cases it will not, but it is not possible to determine this until the needle is actually exposed.

WO 02/066093 A2 (Serpomed Ltd) describes catheter placement devices for peripheral blood vessel catheterization with protected needle tip and shortened length in a transport position. In the transport position, the needle and catheter units are held inside the handle. A user transposes the needle and catheter units into a duty ready position, wherein the needle unit is engaged with the handle distal end and a catheter hub protrudes distally out of the handle. After catheter insertion into a patient's vein, the user disconnects the needle unit and handle allowing needle unit retraction by a resilient member into the protection position.

The present invention is intended to address the problems with the prior art.

### BRIEF SUMMARY OF THE DISCLOSURE

In accordance with a first aspect of the present invention there is provided an introducer needle device, comprising a needle housing, needle, and a resilient means; the needle comprising a needle hub at one end and a beveled needle tip at another end housed at least partially within the needle housing; the needle housing including indicia that comprises a means for identifying an angular orientation of the beveled needle tip within the needle housing; the needle being movable axially along a length of the needle housing between a first position relative to the needle housing in which the needle tip is contained within the needle housing and a second position relative to the needle housing in which at least a part of the needle tip extends beyond a periphery of the needle housing; the resilient means being arranged to bias the needle towards and into the first position and being compressible to enable movement of the needle towards and into the second position; wherein the device further comprises means for preventing rotation of the needle within the needle housing; and the needle housing further comprises a coupling means for coupling the introducer needle device to a separate component, wherein each of the coupling means and the separate component comprises a luer hub fitting the coupling means being arranged relative to the needle such that coupling of the separate component to the coupling means causes the separate component to engage and move the needle hub so that the resilient means are compressed and the needle is moved towards and arranged in the second position.

The needle preferably comprises a hollow bore (lumen) extending between the needle hub and the needle tip.

The needle hub is configured to engage with the separate component. The separate component is capable of moving the needle hub axially within the housing.

The needle housing may comprise an elongate portion for retaining the needle. The needle housing, for example but not limited to the elongate portion, a distal end of the elongate portion may comprise a bevelled tip.

The needle housing may comprise plastic.

The needle housing may comprise metal.

The resilient means may comprise any biasing means such as a biased member that may exert a reactive force when acted upon. In certain embodiments, the resilient means may comprise a spring. Additionally or alternatively, the resilient means may comprise a compressible body or material, including but not limited to rubber, foam or any other compressible material.

At least a part of the resilient means may be housed within at least a part of the needle housing. For example, at least a part of the resilient means may be housed within a hub portion of the housing, and/or the resilient means may be housed within a hub portion and a separate portion of the housing. The separate portion of the housing may be connectable, and/or releasably connectable to the remainder of the housing, for example be a connecting means.

The separate component may be a syringe. The separate component or syringe may comprise a male luer lock or slip to enable the separate component or syringe to be locked onto a part of the introducer needle device. Optionally, the separate component may comprise an end cap. The separate component or syringe may comprise a slip tip for securing it to a part of the introducer needle device.

The separate component or syringe may comprise any connection means, including any shape or configuration of connection means capable of engaging with a corresponding connection means on a part of the introducer needle device.

The means for preventing rotation may comprise one or more channels and/ or one or more projections on the needle housing for engaging with corresponding one or more projections and/ or one or more channels on the needle hub.

The one or more projections may be arranged to slide within the one or more channels. In such a configuration, the needle remains axially movable along the needle housing as the projections slide along the channels, but rotation is prevented by retention of the one or more projections within the one or more channels.

The means for preventing rotation may comprise one or more recesses and/ or one or more projections on the needle housing for engaging with corresponding one or more projections and/ or one or more recesses on the needle hub.

The one or more projections and/or the one or more recesses on each of the needle housing and needle hub may be of any shape or configuration, including but not limited to, an asymmetrical shape or a tab and corresponding releasable locking feature arrangement.

The means for preventing rotation may comprise frictional engagement between the needle hub and the needle housing.

The means for preventing rotation may comprise one or more channels and/ or one or more projections on the coupling means for engaging with corresponding one or more projections and/ or one or more channels on the needle hub.

The means for preventing rotation may comprise one or more recesses or one or more projections on the coupling means for engaging with corresponding one or more projections or one or more recesses on the needle hub.

The one or more projections and/or the one or more recesses on each of the coupling means and needle hub may be of any shape or configuration, including but not limited to, an asymmetrical shape or a tab and corresponding releasable locking feature arrangement.

The means for preventing rotation may comprise frictional engagement between the needle hub and the coupling means.

The means for preventing rotation may comprise a particular geometrical arrangement capable of preventing rotation. For example, the means for preventing rotation may include, but not be limited to, the provision of a needle hub having a polygon configuration and a correspondingly shaped receiving portion on a part of the needle housing.

For example, there may be a square shaped receiving portion on the needle housing for engaging with a corresponding square shaped portion on the needle hub. It is to be appreciated that other polygons and geometric configurations will also be capable of providing substantially the same technical effect, i.e. prevention of rotation of the needle, for example but not limited to, triangular shaped receiving and engaging portions on each of the needle housing and needle hub, or an ellipsoidal needle hub. Therefore, the explicit recitation of square, triangular and other shapes above is not intended to be limiting.

The means for preventing rotation may comprise any combination of the aforementioned features and/or locations of said features on the device.

Each of the coupling means and separate component comprise a luer hub fitting.

The coupling means of the needle housing may comprise a female luer hub fitting and, optionally, the separate component may comprise a male luer hub fitting.

The coupling means may be separable from a part of the needle housing. For example, the coupling means may comprise a proximal portion of the needle housing. The proximal part may house at least a part of the needle hub and/ or at least a part of the resilient means when the device is assembled.

The needle housing may comprise a first part and a second part. The coupling means may define the first part of the needle housing.

A middle and a distal part of the needle housing may house at least a part of the needle hub and/or at least a part of the resilient means, and/or at least a part of the needle.

The middle and/or distal part of the needle housing may define the second part of the needle housing.

The first part and second part of the needle housing may be connectable, for example releasably connectable. In one or more embodiments, the first part and the second part of the needle housing may be permanently coupled after connection of the two parts.

The second part of the needle housing may comprise a housing hub. The housing hub may be an enlarged region of the second part of the needle housing, as compared to the remaining region(s) of the first or second parts of the needle housing.

The housing hub may be arranged to accommodate at least a part of the needle hub and/or at least a part of the resilient means.

At least a part of each of the housing hub and needle hub may be arranged coaxially. For example, the needle hub may sit within a cavity defined by a periphery of the housing hub.

The indicia may, for example, comprise a means of identifying an angular orientation of a bevel of the needle within the housing, for the purpose of orientating the bevel in a required position, e.g. "bevel up" or "bevel down" for insertion into a blood vessel.

In accordance with a second aspect of the present invention there is provided an assembly comprising: an introducer needle device according to any features of the first aspect of the invention, and a separate component arranged to removably connect to a part of the introducer needle device.

The separate component may comprise a connector for connecting the component to the needle housing of the introducer needle device.

The separate component may be connectable to the coupling means of the introducer needle device.

The separate component may comprise a syringe.

The separate component or syringe may comprise a luer connector or a slip tip or any other connection means suitable for coupling to the introducer needle device. It will be appreciated that in other examples or embodiments of the invention, the syringe (or other separate component) and coupling means may not comprise a luer connection, but may be provided with a different connection means, including but not limited to a slip/ push fitting.

It is to be appreciated that examples of materials for the various features of the invention listed herein are not intended to be limiting and that the invention can be put into practice using features comprising any suitable materials. For example, embodiments of the present invention may utilise features comprising materials defined by one or more industry standards or practice.

### BRIEF DESCRIPTION OF THE DRAWINGS

Embodiments of the invention are further described, by way of example, hereinafter with reference to the accompanying drawings, in which:

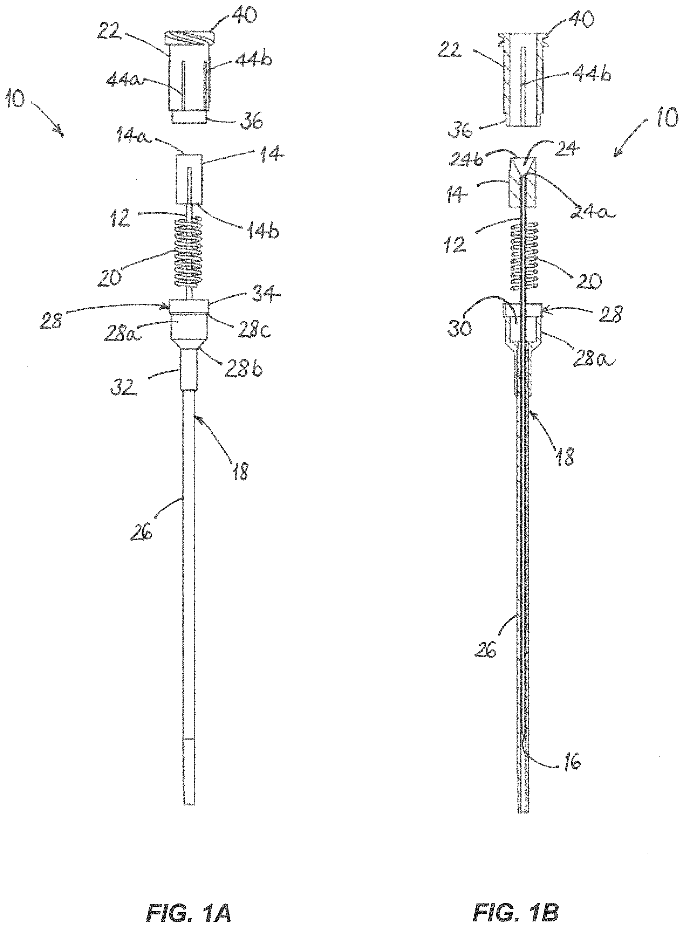

Fig. 1A is an exploded plan view of an embodiment of an introducer needle device according to the invention, with the needle in a retracted, or inactive position;
Fig. 1B is a cross-sectional view of the embodiment shown in Fig. 1A;
Fig. 2A is a plan view of the embodiment of introducer needle device shown in Fig. 1A in an assembled configuration, with the needle in a retracted, or inactive position;
Fig. 2B is a cross-sectional view of the embodiment shown in Fig. 2A;
Fig. 3A is a plan view of the embodiment of introducer needle device shown in Fig. 2A, with the needle in an extended, or active position;
Fig. 3B is a cross-sectional view of the embodiment shown in Fig. 3A;
Fig. 4A is an exploded cross-sectional view of the embodiment shown in Fig. 1A, showing the anti-rotation mechanism of the device in more detail; and
Fig. 4B is a cross-sectional view of the coupling means of the body and the needle hub of the embodiment shown in Fig. 4A showing an enlarged view of the anti-rotation mechanism of the device.

### DETAILED DESCRIPTION

The terms "proximal" and "distal" used herein are used for ease of referencing features of the introducer needle device and are intended only to refer to relative positions of the features, rather than to define a particular orientation of the device. Whilst the meaning of these terms is well known in the art, for the avoidance of doubt it is stated that the term "proximal" is intended to define a feature positioned nearest to the non-bevelled end of the needle, whereas the term "distal" refers to a feature positioned nearest to the bevelled end of the needle.

Referring to the drawings, an embodiment of introducer needle device 10 comprises an elongate needle 12 having a needle hub 14 at a proximal end and a bevel 16 defining the needle tip 16 at a distal end. The device further comprises a needle housing 18 which removably and coaxially surrounds substantially most of the needle 12. A resilient means 20 is positioned between the distal end of the needle hub 14 and the proximal end of the needle housing 18 and is capable of coaxially nesting inside a proximal portion of the housing 18. The device 10 further comprises a coupling means 22 for coupling the device 10 to a separate component during use, for example a syringe (not shown).

The needle 12 comprises an elongate tube-like metal body 12 terminating at the proximal end with a generally cylindrical-shaped plastic hub portion 14. The distal two-thirds of the hub portion 14 comprise a solid plastic core with a centrally located through-bore into which the proximal end of the needle body 12 is secured in a coaxial arrangement, such that the needle hub 14 coaxially surrounds the proximal end of the needle 12. In the present example, when the introducer needle device 10 is assembled ready for use, the needle 12 and needle hub 14 would be bonded together. However, it is to be appreciated that this may not necessarily be the case in one or more other examples or embodiments of the device 10.

The distal end of the needle body 12 terminates in a bevel 16. The needle 12 comprises a central bore or lumen, extending between the proximal and distal ends of the needle body 12. Each of the proximal and distal ends of the needle body 12 are open-ended.

The proximal third of the core of the cylindrical-shaped needle hub portion 14 comprises a generally conical-shaped recess 24, aligned such that the apex 24a of the conical recess 24 is positioned distally within the needle hub portion 14, relative to the wider, generally planar base portion 24b of the conical recess 24, which is positioned on the proximal edge 14a of the hub portion 14. The apex 24a of the conical recess 24 is open and in fluid communication with the central lumen of the adjacent proximal end of the needle 12 which is secured within the core of the hub portion 14 as previously explained. In the present example, there is a small step between the apex 24a and the proximal end of the needle 12 to prevent the needle 12 passing through the hub portion 14. The conical recess 24 is particularly beneficial for funneling a guidewire (not shown) into the lumen of the needle 12. However, this example use is not intended to limit the function of the conical recess 24.

The needle housing 18, which may also be referred to as a needle sheath, comprises an elongate tubular body portion 26 having a bore extending therethrough and is formed from plastic. The needle housing 18 is open at the distal and proximal ends and terminates with a housing hub 28 at the proximal end. The distal end comprises a bevelled tip. The housing hub 28 extends upwardly and outwardly in a tapered manner from the proximal end of the tubular body portion 26 to define a receiving portion which comprises a generally cylindrically shaped enclosing wall 28a. The aforementioned taper ensures a smooth transition of the needle 12 relative to the tubular body portion 26 when the needle is activated. The enclosing wall 28a defines a bore 30 of the hub 28 into which at least a portion of the resilient means 20 can be received, as will be explained. The wider bore 30 of the housing hub is in fluid communication with the narrower bore of the elongate body portion 26, positioned distally adjacent the bore 30 of the hub 28.

The distal end of the cylindrical enclosing wall 28a of the housing hub 28 comprises a distal shoulder region 28b which tapers inwardly and downwardly towards the elongate body portion 26 to converge the wider bore 30 of the housing hub with the narrower bore of the body portion 26. A generally tubular neck portion 32 bridges the shoulder region 28b and the proximal end of the tubular body portion 26. The neck portion 32 comprises a bore which coaxially surrounds the needle 12.

The proximal end of the needle housing hub 28 comprises a proximal shoulder portion 28c which extends proximally from the enclosing wall 28a to define a peripheral lip 34 which forms a female connecting part into which a corresponding male connector on the coupling means 22 can be connected, as will be explained.

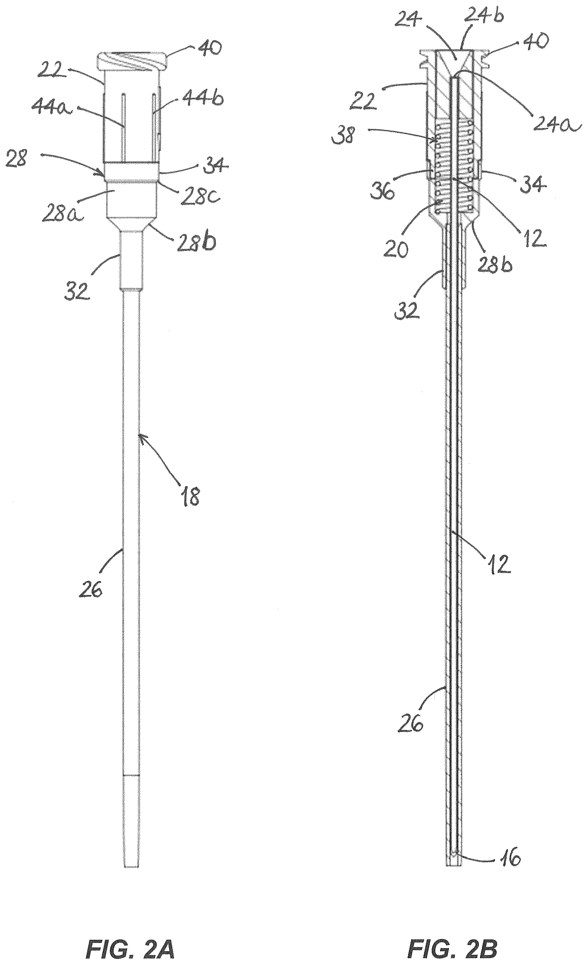

A resilient means 20 in the form of a spring 20 coaxially surrounds a proximal region of the needle 12 and is arranged to removably sit in the bore 30 of the housing hub 28. In an uncompressed configuration, as best seen in Fig. 2, a distal half of the spring 20 sits in the bore 30 of the housing hub 28, and a proximal half extends upwardly beyond the peripheral lip 34 of the housing hub 28, to abut a distal edge of the needle hub 14.

The coupling means 22 forms a part of the needle housing 18, specifically in the present example, a part of the housing hub 28, and comprises a generally cylindrical-shaped push-fit style connector, having a hollow elongate body comprising a central bore. The coupling means 22 terminates at a distal edge with a male connector 36 adapted to frictionally engage with a corresponding female connector defined by the peripheral lip 34 of the housing hub 28. When the male and female connectors 36, 34 are coupled together, the respective bores 30 of the male and female connectors 36, 34 are brought together and are in fluid communication with one another to form a cavity 38 within a proximal portion of the device 10. It will be appreciated that in other examples or embodiments of the device, the coupling means 22 and housing hub 28 could comprise complementary screw-threads to enable the aforementioned male and female connectors 36, 34 to be screwed together.

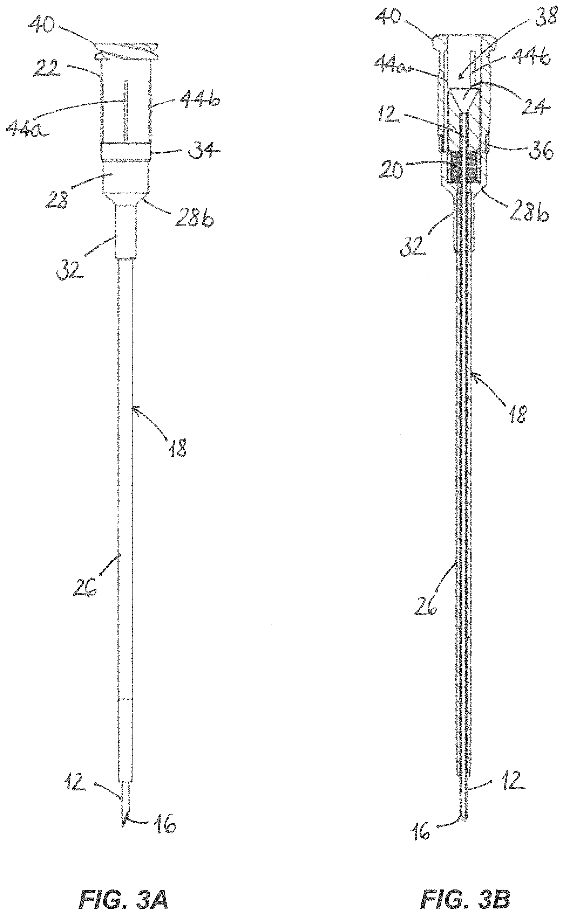

When the male and female connectors 36, 34 are coupled together, the spring 20, needle hub 14 and proximal portion of the needle 12, are all securely contained within the cavity 38, as best seen in Figs. 2B and 3B.

In the present embodiment, the proximal peripheral edge of the coupling means 22 comprises a female luer lock connector 40, as visible in the drawings. The female luer lock 40 is adapted to connect to a corresponding male luer lock on a syringe (not shown). In the present embodiment, a 6% bevel is provided on the internal surface of the proximal peripheral edge of the coupling means 22 to facilitate connection of a luer slip fitting, in accordance with industry standards. However, it will be appreciated that other examples or embodiments of the device 10 may not comprise this 6% bevel, or may be provided with a different bevel configuration, and so the aforementioned disclosure is not intended to be limiting with respect to the present invention.

Figs. 2A and 2B show the device 10 in an inactivated configuration, i.e. in a configuration in which it would be received by end an user such as a clinician. As is best seen in Fig. 2B, in this configuration, the coupling means 22 is coupled to the housing hub 28 to create the aforementioned cavity 38, in which each of a part of the needle 12 and spring 20 are coaxially arranged.

In this inactivated configuration, the proximal edge 14a of the needle hub 14 is aligned with the peripheral proximal edge of the coupling means 22, defined in the present embodiment as a female luer connector 40. This arrangement provides more space within the cavity 38 into which the spring 20 can expand, as compared to the activated configuration shown in Figs. 3A and 3B.

As the spring 20 is in an expanded configuration and substantially fills the cavity 38, it is biasing the needle hub 14 towards the proximal edge 40 of the coupling means 22, meaning that the needle 12 which is secured to the needle hub 14 is fully enclosed within the needle housing 18 as best seen in Fig. 2B. This biasing arrangement retains the bevelled needle tip 16 within the housing 18 to prevent needle stick injuries, thus providing a safe introducer needle device 10.

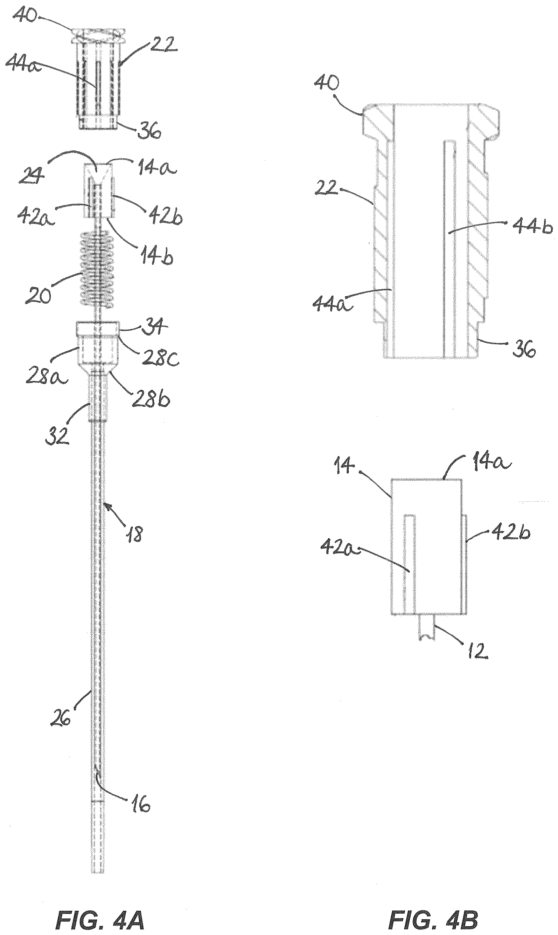

In the inactivated configuration, an anti-rotation means is engaged to prevent rotation of the needle 12 within the needle housing 18, as will be explained. However, the anti-rotation means may also prevent rotation of the needle 12 in any configuration (i.e. active or inactive) of the device. As most clearly seen in Figs. 4A and 4B, in the present embodiment, the anti-rotation means comprises three spaced apart elongate projections (only two of which are visible in Fig. 4B) 42a, 42b disposed on the outer peripheral surface of the needle hub 14 and three correspondingly shaped and sized channels (only two of which are visible on Fig. 4B) 44a, 44b on the interior peripheral surface of the coupling means 22 of the needle housing 18. The three elongate projections 42a, 42b (third not visible) extend radially outward from the outer peripheral surface of the needle hub 14.

Each projection 42a, 42b is arranged perpendicularly with respect to the proximal and distal edges 14a, 14b of the needle hub 14. Each projection 42a, 42b extends proximally from the distal edge 14b of the needle hub 14 to approximately 7/8 the length of the needle hub 14, terminating just distally of the proximal edge 14a of the needle hub 14. The projections 42a, 42b are adapted to slide axially in the correspondingly shaped and sized channels 44a, 44b located on the inner surface of the coupling means 22, as best seen in Fig. 4B.

Each channel 44a, 44b comprises an elongate recess 44a, 44b in the inner peripheral surface of the coupling means 22 of the needle housing 18. The three channels 44a, 44b (third channel not visible) are spaced apart to the same extent as the corresponding projections 42a, 42b (third projection not visible) on the needle hub 14, so that when the needle hub 14 is coaxially inserted into the coupling means 22 of the needle housing 18, the channels 44a, 44b and projections 42a, 42b can be aligned and engaged with one another as the coupling means 22 is slid over the needle hub 14. This arrangement enables the needle hub 14, and therefore the needle 12, to slide axially within the needle housing 18 whilst preventing rotation of the needle hub 14 and needle 12 relative to the needle housing 18. In at least the present example of the device 10, the channels 44a, 44b and projections 42a, 42b are always at least partially engaged with one another, meaning that rotation of the needle 12 is prevented at all times, irrespective of whether the device 10 is in an active or inactive configuration.

As seen clearly in the Figures, the channels 44a, 44b do not extend the length of the coupling means 22, but rather, extend proximally from the distal edge of the coupling means 22, just above the male connector 36, substantially three quarters the length of the coupling means 22 such that approximately the proximal quarter of the coupling means 22 does not have any channels. This arrangement prevents the needle hub 14 from being pushed out of the proximal opening 40 of the coupling means 22 by the force of the spring 22.

This anti-rotation means is particularly useful at least because it ensures that the bevel 16 of the needle 12 is aligned as intended during manufacture and assembly of the device 10, so that its position within the housing 18 can be immediately determined by the end user even when the bevel 16 is retained in the housing 18.

This determination can be assisted for example by means of an indicator (not shown) on the outer surface of the housing 18 which indicates the position of the bevel 16 within the housing 18. Typically, the indicator takes the form of an arrow. The end user (usually a clinician) can then correctly align the device 10 for insertion into a patient's blood vessel without having to first expose the bevel 16 from the housing and subsequently rotate it to correctly position the bevel 16 for use.

During assembly of the device 10, which is typically conducted by a device manufacturer, the spring 20 is threaded onto the body of the needle 12 and positioned at the proximal end of the needle 12 adjacent the needle hub 14. The spring 20 therefore coaxially surrounds the proximal end of the metal body portion of the needle 12.

The needle 12 is inserted into the elongate body of the needle housing 18 via the peripheral lip 34 of the housing hub 28, thus positioning the spring 20 between the housing hub 28 and the needle hub 14. Specifically, the distal end of the spring 20 is seated within the central bore 30 of the housing hub 28 whilst the proximal end of the spring 20 abuts the distal edge 14b and underside of the needle hub 14.

The coupling means 22 is aligned so as to align the elongate projections 42a, 42b on the needle hub 14 with the corresponding elongate channels 44a, 44b on the coupling means 22. Once aligned, the coupling means 22 is slid over the outer periphery of the needle hub 14 and distally towards the housing hub 28, so that the coupling means 22 coaxially surrounds the needle hub 14 and the distal edge 36, which defines the male connector 36 of the coupling means 22, is push-fitted into the proximal edge 34 which defines the female connector 34, of the needle hub 14. Once the coupling means 22 is slid into position and the male and female connectors 36, 34 are coupled together to close the device 10 into its assembled position, the spring 20 is securely positioned in an uncompressed configuration within the cavity 38 between the needle hub 14 and the housing hub 28.

To use the introducer needle device 10, the bevel 16 of the needle 12 and a distal region of the needle 12 need to be extended outwardly from the bore and beyond the distal edge of the needle housing 18, as shown in Figs. 3A and 3B. This can be achieved by coupling a male luer connector of a syringe (not shown) to the female luer connector 40 on the coupling means 22.

The distal tip of the male luer connector (not shown) engages with the conical recess 24 of the needle hub 14 and fits within the enclosing wall of the coupling means 22. Specifically, the male luer connector further comprises a screw-threaded lock portion (not shown) corresponding to the screw-threaded lock portion of the female luer connector 40, such that the two can be secured together, in a manner typical for this known type of connector. As the male luer connector is introduced into the coupling means 22 for connection, it engages with proximal end 14a of the needle hub 14 and pushes it distally towards the distal end of the housing 24 as it is connected to the female luer connector 40 of the coupling means 22, which moves the needle 12 away from the inactivated position and towards the activated position ready for use. It will be appreciated that in other examples or embodiments of the invention, the syringe (or other type of separate component) and coupling means may not comprise a luer connection, but may instead be provided with a different connection means, including but not limited to a slip/ push fitting.

As the needle hub 14 is moved towards the activated position, i.e. distal end of the housing, the spring 20 becomes compressed such that when the male luer connector and female connecting portion 40 are coupled together, the spring 20 is fully compressed and retained within the enclosing wall 28a of the housing hub 28, as best seen in Fig. 3B, instead of extending between the enclosing wall 28a of the housing hub 28 and substantially half way into the bore of the coupling means 22.

Compression of the spring 20 overcomes the bias from the extended spring 20 that was previously acting on the needle 12 to retain it within the needle housing 18. Therefore, with the spring in a compressed configuration, the needle 12, in particular the distal end of the needle 12 including the bevel 16, extends beyond the distal edge of the elongate body portion 26 of the housing 18 ready for use, as best seen in Figs. 3A and 3B. The bevel 16 and needle tip remain exposed from the body portion 26 of the housing 18 until the syringe and coupling means 22 are disconnected from one another.

Disconnection of the syringe from the coupling means 22 of the device 10 allows the spring 20 to decompress, which draws the needle hub 14 in a proximal direction towards the proximal edge of the coupling means 22. As a consequence, the needle 12 attached to the needle hub 14 is drawn in a proximal direction and into the elongate body portion 26 of the needle housing 18 to move the needle 12 back towards the inactivated position. Therefore, as the spring 20 decompresses, the needle 12 becomes biased back towards the retracted position, as best seen in Figs. 2A and 2B.

Although activation of the device 10 is described in the present example as being initiated by coupling of a syringe, it is to be appreciated that the introducer needle device 10 can be initiated or activated by insertion or coupling of any component or device capable of initiating the device by causing compression of the resilient means 20 of the device.

It will be appreciated that the embodiment described herein relates to a non-limiting example of an embodiment of the invention and that, as such, other configurations or arrangements of introducer needle device may be suitable whilst still falling under the scope of the claimed invention. For example, the anti-rotation means may comprise an alternative configuration to that described with reference to the example embodiment herein. For example, the anti-rotation means may comprise any arrangement of complementarily shaped projections and recesses positioned on each of the needle hub and coupling means, and/or one or more tabs and corresponding releasable locking features on each of the needle hub and coupling means, and/or frictional engagement between the needle hub and the coupling means. By way of further example, the anti-rotation means may comprise a particular shape of needle hub and/ or coupling means that prevents rotation of the needle, such as but not limited to a square shaped needle hub.

Throughout the description and claims of this specification, the words "comprise" and "contain" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

Modifications may be performed on the basis of the general knowledge of the skilled person within the scope of the appended claims.

## Claims

1. An introducer needle device (10), comprising a needle housing (18), a needle (12), and a resilient means (20);
the needle (12) comprising a needle hub (14) at one end and a bevelled needle tip (16) at another end housed at least partially within the needle housing (18);
the needle housing (18) including indicia that comprises a means for identifying an angular orientation of the bevelled needle tip (16) within the needle housing (18);
the needle (12) being movable axially along a length of the needle housing (18) between a first position relative to the needle housing (18) in which the needle tip (16) is contained within the needle housing (18) and a second position relative to the needle housing (18) in which at least a part of the needle tip (16) extends beyond a periphery of the needle housing (18);
the resilient means (20) being arranged to bias the needle (12) towards and into the first position and being compressible to enable movement of the needle (12) towards and into the second position;
wherein the device (10) further comprises means for preventing rotation of the needle (12) within the needle housing (18); and
the needle housing (18) further comprises a coupling means (22) for coupling the introducer needle device (10) to a separate component, wherein each of the coupling means (22) and the separate component comprises a luer hub fitting, the coupling means (22) being arranged relative to the needle (12) such that coupling of the separate component to the coupling means (22) causes the separate component to engage and move the needle hub (14) so that the resilient means (20) are compressed and the needle (12) is moved towards and arranged in the second position.

2. An introducer needle device (10) as claimed in claim 1, wherein the resilient means (20) comprises a spring.

3. An introducer needle device (10) as claimed in any of claims 1 to 2, wherein at least a part of the resilient means (20) is housed within at least a part of the needle housing (18).

4. An introducer needle device (10) as claimed in any preceding claim, wherein the coupling means (22) of the needle housing (18) comprises a female luer fitting (40), and, optionally, the separate component comprises a male luer hub fitting.

5. An introducer needle device (10) as claimed in any preceding claim, wherein the means for preventing rotation comprises one or more channels (44a, 44b) or projections (42a, 42b) on the needle housing (18) for engaging with corresponding one or more projections (42a, 42b) or channels (42a, 42b) on the needle hub (14).

6. An introducer needle device (10) as claimed in any preceding claim, wherein the means for preventing rotation comprises frictional engagement between the needle hub (14) and the needle housing (18).

7. An introducer needle device (10) as claimed in any preceding claim, wherein the means for preventing rotation comprises a needle hub (14) having a polygon configuration and a correspondingly shaped receiving portion on a part of the needle housing (18).

8. An introducer needle device (10) as claimed in any preceding claim, wherein the needle housing (18) comprises a first part and a second part, and, optionally, wherein the first part and the second part of the needle housing (18) are connectable to one another.

9. An introducer needle device (10) as claimed in claim 8 wherein the second part comprises the coupling means (22).

10. An assembly comprising: an introducer needle device (10) according to any preceding claim, and a separate component comprising a luer hub fitting is arranged to removably connect to a part of the introducer needle device (10).

11. An assembly as claimed in claim 10, wherein the separate component comprises a connector for connecting the separate component to the needle housing (18) of the introducer needle device (10).

12. An assembly as claimed in claim 10 or claim 11, wherein the separate component is connectable to the coupling means (22) of the introducer needle device (10).

13. An assembly according to claim 12, wherein the separate component comprises a syringe.

## Patentansprüche

1. Einführnadelvorrichtung (10), umfassend ein Nadelgehäuse (18), eine Nadel (12),
und eine elastische Einrichtung (20);
die Nadel (12) umfassend an einem Ende eine Nadelnabe (14) und an einem anderen Ende eine abgeschrägte Nadelspitze (16), die zumindest teilweise in dem Nadelgehäuse (18) untergebracht ist,
wobei das Nadelgehäuse (18) eine Kennzeichnung beinhaltet, die eine Einrichtung zum Identifizieren einer winkligen Ausrichtung der abgeschrägten Nadelspitze (16) in dem Nadelgehäuse (18) umfasst,
wobei die Nadel (12) entlang einer Länge des Nadelgehäuses (18) zwischen einer ersten Position in Bezug auf das Nadelgehäuse (18), in der die Nadelspitze (16) in dem Nadelgehäuse (18) enthalten ist, und einer zweiten Position in Bezug auf das Nadelgehäuse (18), in der sich zumindest ein Teil der Nadelspitze (16) über einen Umfang des Nadelgehäuses (18) hinaus erstreckt, axial bewegbar ist,
wobei die elastische Einrichtung (20) angeordnet ist, um die Nadel (12) zu der und in die erste Position vorzuspannen, und komprimierbar ist, um eine Bewegung der Nadel (12) zu der und in die zweite Position zu ermöglichen;
wobei die Vorrichtung (10) ferner Einrichtungen zum Verhindern einer Drehung der Nadel (12) in dem Nadelgehäuse (18) umfasst, und
das Nadelgehäuse (18) ferner eine Kopplungseinrichtung (22) zum Koppeln der Einführnadelvorrichtung (10) mit einer separaten Komponente umfasst, wobei sowohl die Kopplungseinrichtung (22) und die separate Komponente einen Luer-Nabenanschluss umfassen, wobei das Kopplungseinrichtung (22) in Bezug auf die Nadel (12) angeordnet ist, sodass ein Koppeln der separaten Komponente mit der Kopplungseinrichtung (22) bewirkt, dass die separate Komponente die Nadelnabe (14) eingreift und diese bewegt, sodass die elastischen Einrichtung (20) zusammengedrückt wird und die Nadel (12) zu der zweiten Position bewegt wird und dort angeordnet ist.

2. Einführnadelvorrichtung (10) nach Anspruch 1, wobei die elastische Einrichtung (20) eine Feder umfasst.

3. Einführnadelvorrichtung (10) nach einem der Ansprüche 1 bis 2, wobei zumindest ein Teil der elastischen Einrichtung (20) in zumindest einem Teil des Nadelgehäuses (18) untergebracht ist.

4. Einführnadelvorrichtung (10) nach einem vorherigen Anspruch, wobei die Kopplungseinrichtung (22) des Nadelgehäuses (18) eine Luer-Anschlussbuchse (40) umfasst und optional die separate Komponente einen Luer-Nabenanschlussstecker umfasst.

5. Einführnadelvorrichtung (10) nach einem vorherigen Anspruch, wobei die Einrichtung zum Verhindern einer Drehung einen oder mehrere Kanäle (44a, 44b) oder Vorsprünge (42a, 42b) an dem Nadelgehäuse (18) zum Eingreifen mit einem oder mehreren entsprechenden Vorsprüngen (42a, 42b) oder Kanälen (42a, 42b) an der Nadelnabe (14) umfasst.

6. Einführnadelvorrichtung (10) nach einem vorherigen Anspruch, wobei die Einrichtung zum Verhindern einer Drehung einen Reibungseingriff zwischen der Nadelnabe (14) und dem Nadelgehäuse (18) umfasst.

7. Einführnadelvorrichtung (10) nach einem vorherigen Anspruch, wobei die Einrichtung zum Verhindern einer Drehung eine Nadelnabe (14), die eine Polygonkonfiguration aufweist, und einen entsprechend geformten Aufnahmeabschnitt an einem Teil des Nadelgehäuses (18) umfasst.

8. Einführnadelvorrichtung (10) nach einem vorherigen Anspruch, wobei das Nadelgehäuse (18) einen ersten Teil und einen zweiten Teil umfasst, und optional wobei der erste Teil und der zweite Teil des Nadelgehäuses (18) miteinander verbunden werden können.

9. Einführnadelvorrichtung (10) nach Anspruch 8, wobei der zweite Teil die Kopplungseinrichtung (22) umfasst.

10. Anordnung, umfassend: eine Einführnadelvorrichtung (10) nach einem vorherigen Anspruch und eine separate Komponente, umfassend einen Luer-Nabenanschluss, der angeordnet ist, um lösbar mit einem Teil der Einführnadelvorrichtung (10) verbunden zu sein.

11. Anordnung nach Anspruch 10, wobei die separate Komponente einen Verbinder zum Verbinden der separaten Komponente mit dem Nadelgehäuse (18) der Einführnadelvorrichtung (10) umfasst.

12. Anordnung nach Anspruch 10 oder Anspruch 11, wobei die separate Komponente mit der Kopplungseinrichtung (22) der Einführnadelvorrichtung (10) verbunden werden kann.

13. Anordnung nach Anspruch 12, wobei die separate Komponente eine Spritze umfasst.

## Revendications

1. Dispositif d'aiguille d'introducteur (10), comprenant un logement d'aiguille (18), une aiguille (12) et un moyen élastique (20) ;
l'aiguille (12) comprenant un raccord d'aiguille (14) au niveau d'une extrémité et une pointe d'aiguille biseautée (16) au niveau d'une autre extrémité logée au moins partiellement dans le logement d'aiguille (18) ;
le logement d'aiguille (18) comprenant des repères qui comprennent un moyen permettant d'identifier l'orientation angulaire de la pointe d'aiguille biseautée (16) à l'intérieur du logement d'aiguille (18) ;
l'aiguille (12) étant mobile axialement le long d'une longueur du logement d'aiguille (18) entre une première position par rapport au logement d'aiguille (18) dans laquelle la pointe d'aiguille (16) est contenue dans le logement d'aiguille (18) et une seconde position par rapport au logement d'aiguille (18) dans laquelle au moins une partie de la pointe d'aiguille (16) s'étend au-delà de la périphérie du logement d'aiguille (18) ;
le moyen élastique (20) étant agencé pour solliciter l'aiguille (12) vers et dans la première position et étant compressible pour permettre le déplacement de l'aiguille (12) vers et dans la seconde position ;
ledit dispositif (10) comprenant en outre un moyen permettant d'empêcher la rotation de l'aiguille (12) à l'intérieur du logement d'aiguille (18) ; et
ledit logement d'aiguille (18) comprenant en outre un moyen de couplage (22) permettant de coupler le dispositif d'aiguille d'introducteur (10) à un composant distinct, chacun parmi le moyen de couplage (22) et le composant distinct comprenant un embout de raccord Luer, ledit moyen de couplage (22) étant agencé par rapport à l'aiguille (12) de sorte que le couplage du composant distinct au moyen de couplage (22) amène le composant distinct à se mettre en prise avec le raccord d'aiguille (14) et à le déplacer de sorte que le moyen élastique (20) soit comprimé et que l'aiguille (12) soit déplacée vers et disposée dans la seconde position.

2. Dispositif d'aiguille d'introducteur (10) selon la revendication 1, ledit moyen élastique (20) comprenant un ressort.

3. Dispositif d'aiguille d'introduceur (10) selon l'une quelconque des revendications 1 à 2, au moins une partie du moyen élastique (20) étant logée dans au moins une partie du logement d'aiguille (18).

4. Dispositif d'aiguille d'introducteur (10) selon l'une quelconque des revendications précédentes, ledit moyen de couplage (22) du logement d'aiguille (18) comprenant un embout Luer femelle (40) et, éventuellement, ledit composant distinct comprenant un embout de raccord Luer mâle.

5. Dispositif d'aiguille d'introducteur (10) selon l'une quelconque des revendications précédentes, ledit moyen permettant d'empêcher la rotation comprenant un ou plusieurs canaux (44a, 44b) ou saillies (42a, 42b) sur le logement d'aiguille (18) destinés à se mettre en prise avec une ou plusieurs plusieurs saillies (42a, 42b) ou un ou plusieurs canaux (42a, 42b) correspondants sur le raccord d'aiguille (14).

6. Dispositif d'aiguille d'introducteur (10) selon l'une quelconque des revendications précédentes, ledit moyen permettant d'empêcher la rotation comprenant une mise en prise par frottement entre le raccord d'aiguille (14) et le logement d'aiguille (18).

7. Dispositif d'aiguille d'introducteur (10) selon l'une quelconque des revendications précédentes, ledit moyen permettant d'empêcher la rotation comprenant un raccord d'aiguille (14) comportant une configuration polygonale et une partie de réception formée en conséquence sur une partie du logement d'aiguille (18).

8. Dispositif d'aiguille d'introducteur (10) selon l'une quelconque des revendications précédentes, ledit logement d'aiguille (18) comprenant une première partie et une seconde partie, et, éventuellement, ladite première partie et ladite seconde partie du logement d'aiguille (18) pouvant être reliées l'une à l'autre.

9. Dispositif d'aiguille d'introducteur (10) selon la revendication 8, ladite seconde partie comprenant le moyen de couplage (22).

10. Ensemble comprenant : un dispositif d'aiguille d'introducteur (10) selon l'une quelconque des revendications précédentes, et un composant distinct comprenant un embout de raccord Luer qui est agencé pour être relié de manière amovible à une partie du dispositif d'aiguille d'introducteur (10).

11. Ensemble selon la revendication 10, ledit composant distinct comprenant un élément de raccordement permettant de relier le composant distinct au logement d'aiguille (18) du dispositif d'aiguille d'introducteur (10).

12. Ensemble selon la revendication 10 ou la revendication 11, ledit composant distinct pouvant être relié au moyen de couplage (22) du dispositif d'aiguille d'introducteur (10).

13. Ensemble selon la revendication 12, ledit composant distinct comprenant une seringue.
